# FASCICULE DE BREVET EUROPEEN

(11) **EP 3 173 065 B1**
(45) Date de publication et mention de la délivrance du brevet: **28.02.2018**
(21) Numéro de dépôt: 16200796.7
(22) Date de dépôt: 25.11.2016
(51) Int. Cl.: A61K 8/9789, A61Q 19/08, A61Q 19/02, A61K 36/28

(54) **EXTRAIT ALCOOLIQUE DE PARTIES AÉRIENNES DE SOLIDAGO VIRGAUREA SUBSP. ALPESTRIS, SON PROCÉDÉ D'OBTENTION, ET COMPOSITION COSMÉTIQUE OU DERMATOLOGIQUE LE CONTENANT**
ALKOHOLISCHER EXTRAKT DER OBERIRDISCHEN TEILE VON SOLIDAGO VIRGAUREA SUBSP. ALPESTRIS, DESSEN HERSTELLUNGSPROZESS UND KOSMETISCHE ODER DERMATOLOGISCHE ZUSAMMENSETZUNG DIESEN ENTHALTEND
ALCOHOLIC EXTRACT OF AERIAL PARTS OF SOLIDAGO VIRGAUREA SUBSP. ALPESTRIS, PROCESS OF PREPARATION THEREOF, AND COSMETIC OR DERMATOLOGICAL COMPOSITION COMPRISING SUCH AN EXTRACT

(30) Priorité: 26.11.2015 FR 1561400
(43) Date de publication de la demande: 31.05.2017
(73) Titulaire: Chanel Parfums Beauté, 92200 Neuilly-sur-Seine (FR); CNRS Centre National de la Recherche Scientifique, 75016 Paris (FR); Université De Nice Sophia Antipolis, 06103 Nice (FR)
(72) Inventeur: HOLDERITH, Serge, 94300 VINCENNES (FR); TROMEUR, Anais, 75003 PARIS (FR); BERLIN, Irina, 94300 VINCENNES (FR); FERNANDEZ, Xavier, 06100 NICE (FR); CASALE, Alexandre, 06100 NICE (FR); GRILLARI, Johannes, 2102 Bisamberg (AT); LÄMMERMANN, Ingo, 1170 Wien (AT); GRUBER, Florian, A-1090 Wien (AT); NARZT, Marie-Sophie, 1140 Vienna (AT)
(74) Mandataire: Cabinet Plasseraud

(56) Documents cités:
- WO-A1-2010/072923
- US-A1- 2009 004 302
- US-A1- 2009 214 677
- Lucas Meyer Cosmetics: "Songa (TM) Firming and Regenerating Active", , 9 juillet 2014 (2014-07-09), XP002758558, Extrait de l'Internet: URL:https://web.archive.org/web/2014070901 5925/http://lucasmeyercosmetics.com/en/pro ducts/product.php?id=52&from=cat [extrait le 2016-06-08]
- M. CHEVALIER ET AL: "Inhibition of Candida albicans yeast-hyphal transition and biofilm formation by Solidago virgaurea water extracts", JOURNAL OF MEDICAL MICROBIOLOGY., vol. 61, no. Pt_7, 15 mars 2012 (2012-03-15), pages 1016-1022, XP055278723, GB ISSN: 0022-2615, DOI: 10.1099/jmm.0.041699-0

## Description

L'invention concerne un extrait de parties aériennes de *Solidago virgaurea subsp. alpestris*, son procédé d'obtention, une composition cosmétique ou dermatologique le contenant, ainsi que différentes utilisations cosmétiques.

La peau est principalement constituée de trois couches, à savoir, en partant de la plus superficielle, l'épiderme, le derme et l'hypoderme.

L'épiderme contribue largement à assurer la protection de la peau et à en maintenir son bon fonctionnement.

Le vieillissement et le photo-vieillissement de la peau et les altérations qui y sont associées peuvent se manifester de différentes manières, parmi lesquelles on peut citer :
- la perte de fermeté et d'élasticité dues à une perte tissulaire au niveau de l'épiderme et/ou du derme ;
- la perte d'éclat due à la réduction de la microcirculation et à un ralentissement du renouvellement cellulaire au niveau de l'épiderme;
- l'apparition de taches pigmentaires; et/ou
- la sécheresse cutanée résultant d'une diminution de la fonction barrière de la couche cornée et d'un ralentissement du renouvellement épidermique.

Il existe, de ce fait, un besoin de fournir un agent actif polyfonctionnel susceptible d'agir sur un ensemble de causes d'altérations de la peau dues au vieillissement et/ou à une modification des mécanismes physiologiques liés au vieillissement.

Or, la Demanderesse a maintenant trouvé qu'un extrait alcoolique de parties aériennes de *Solidago virgaurea subsp. alpestris*, obtenu par un procédé particulier, présente, par le biais de stimulation ou d'inhibition de mécanismes physiologiques, des activités intéressantes vis-à-vis du vieillissement cutané, et de la pigmentation et de la microcirculation de la peau. En effet, comme démontré en exemples, l'extrait alcoolique de parties aériennes de *Solidago virgaurea subsp. alpestris* selon l'invention présente des propriétés cosmétiques intéressantes : il permet de lutter contre le vieillissement de la peau, notamment par ses actions anti-MMP3 et anti-MMP9 ; présente des propriétés antioxydantes ; active la microcirculation cutanée ; et présente une activité dépigmentante. L'extrait selon l'invention présente en outre un effet anti-vieillissement de la peau, car il permet de retarder la sénescence réplicative, empêche la transition des fibroblastes papillaires en fibroblastes réticulaires, et réduit l'expression de marqueurs de sénescence.

L'invention concerne donc, selon un premier aspect, un extrait alcoolique de parties aériennes de *Solidago virgaurea subsp. alpestris*, susceptible d'être obtenu par un procédé comprenant les étapes suivantes :
a) extraction des parties aériennes de *Solidago virgaurea subsp. alpestris*, avec au moins un solvant alcoolique ;
b) incubation du mélange obtenu en a) pendant au moins 10h ;
c) filtration du mélange incubé obtenu en b) ; et
d) élimination du solvant du filtrat obtenu, puis dilution finale dans un autre solvant alcoolique.
Un tel extrait est ainsi appelé, dans la présente demande, extrait selon l'invention.

L'invention se rapporte également à un procédé d'extraction de parties aériennes de *Solidago virgaurea subsp. alpestris*, comprenant les étapes suivantes :
a) extraction des parties aériennes de *Solidago virgaurea subsp. alpestris*, avec au moins un solvant alcoolique ;
b) incubation du mélange obtenu en a) pendant au moins 10h ;
c) filtration du mélange incubé obtenu en b) ; et
d) élimination du solvant du filtrat obtenu, puis dilution finale dans un autre solvant alcoolique.

L'invention se rapporte également à une composition cosmétique ou dermatologique comprenant, dans un véhicule cosmétiquement ou pharmaceutiquement acceptable, un extrait alcoolique de parties aériennes de *Solidago virgaurea subsp. alpestris* selon l'invention. Par véhicule cosmétiquement ou pharmaceutiquement acceptable, on entend un milieu compatible avec la peau, les muqueuses et les phanères. De préférence, la composition cosmétique ou dermatologique selon l'invention est adaptée à une application par voie topique.

Le procédé d'obtention de l'extrait selon l'invention comprend ainsi les étapes suivantes :
a) extraction des parties aériennes de *Solidago virgaurea subsp. alpestris*, avec au moins un solvant alcoolique ;
b) incubation du mélange obtenu en a) pendant au moins 10h ;
c) filtration du mélange incubé obtenu en b) ; et
d) élimination du solvant du filtrat obtenu, puis dilution finale dans un autre solvant alcoolique.

La matière première mise en oeuvre est constituée des parties aériennes de *Solidago virgaurea subsp. alpestris.*

*Solidago virgaurea subsp. alpestris* est une plante à fleurs jaunes de la famille des Asteracées, que l'on trouve notamment en région Provence Alpes Côte d'Azur. Le genre Solidago fait partie de la famille des Asteraceae (Asteracées). Il comporte de 156 à 377 espèces, dont l'espèce *Solidago virgaurea L,* communément appelée solidage, verge d'or ou gerbe d'or. La sous-espèce *Solidago virgaurea subsp. alpestris* (Waldst. & Kit.) Gremli, nommé Petit Solidage, Petite Verge-d'or ou encore Solidage alpestre, a pour synonyme *Solidago alpestris* Waldst. & Kit.
C'est une plante herbacée vivace, à tiges fleuries dressées violacées. Les feuilles sont plus ou moins couvertes de poils, simples, alternes, ovales et lancéolées, avec un bord denté. Les fleurs jaunes sont regroupées en capitules possédant 6 à 12 fleurs, eux-mêmes disposés en grappe. La floraison a lieu de juillet à septembre. Les fruits sont des akènes cylindriques jaunâtres.
La sous-espèce *alpestris* se distingue de l'espèce principale *Solidago virgaurea* par sa petite taille (5 à 30 cm au lieu de 1m), le petit nombre des capitules, qui sont par ailleurs un peu plus grands, et par le fait qu'elle pousse plus haut en altitude. La sous-espèce *Solidago virgaurea subsp. alpestris* apparaît en fin de saison dans les éboulis et pentes rocailleuses. Elle pousse en altitude entre 1400 à 2800 mètres dans les régions montagneuses de l'Europe du Nord.

Il existe d'autres sous-espèces, différentes de la sous-espèce d'intérêt selon l'invention. Notamment, on trouve le *Solidago virgaurea subsp. asiatica* Kitam. ex Hara, ayant pour synonymes *Solidago japonica var. japonica* et *Solidago japonica* Kitam. Cette sous-espèce *Solidago virgaurea subsp. asiatica* Kitam. ex Hara mesure 35 à 85 cm et fleurit d'août à novembre. Elle est distribuée au Japon, Corée, Russie, Chine et Philippines.

Les parties aériennes de *Solidago virgaurea subsp. alpestris* utilisées selon l'invention sont typiquement choisies parmi les fleurs, les feuilles, les tiges et leurs mélanges. De préférence, les parties aériennes utilisées sont un mélange de fleurs, feuilles et tiges de *Solidago virgaurea subsp. alpestris.* De préférence, ces parties aériennes sont préalablement séchées, puis broyées ou réduites en morceaux de manière usuelle.
Dans l'étape a), les parties aériennes sont soumises à une extraction par un ou plusieurs solvants alcooliques, par exemple choisis parmi :
- les monoalcools en C₁-C₄, tels que par exemple le méthanol, l'éthanol ou l'isopropanol ; et
- les diols, tels que par exemple le propylène glycol, le 1,3-propanediol ou le dipropylène glycol.
De préférence, le solvant alcoolique est un monoalcool comprenant de 2 à 4 atomes de carbone, plus préférentiellement l'éthanol.
L'extraction est généralement réalisée en immergeant ou en agitant doucement les parties aériennes dans un ou plusieurs des solvants cités ci-dessus à des températures allant, par exemple, de la température ambiante à 80°C, pendant une durée d'environ 30 minutes à 8 h. De préférence, l'extraction de l'étape a) est réalisée pendant une durée comprise entre 2 et 6 heures, à une température comprise entre 40°C et 60°C.

Le mélange obtenu à l'étape a) est ensuite incubé pendant au moins 10h: c'est l'étape b). De préférence, l'incubation de l'étape b) est réalisée pendant une durée comprise entre 12h et 30h, à une température comprise entre 2°C et 10°C. Plus préférentiellement, l'incubation est effectuée pendant 12h à 15h, à une température d'environ 4°C.

Le mélange incubé obtenu à l'issue de l'étape b) est alors filtré afin d'éliminer les substances insolubles : c'est l'étape c). De préférence, la filtration de l'extrait obtenu en b) est effectuée sur une membrane de 100 µm. On obtient ainsi un filtrat liquide.

Enfin, le solvant présent dans le filtrat liquide est éliminé, puis le reste de filtrat est dilué dans un autre solvant alcoolique : c'est l'étape d). Le solvant alcoolique utilisé dans l'étape d) est appelé « autre solvant alcoolique », car il est différent du solvant alcoolique utilisé dans l'étape a). Tenant compte de cette limitation, le solvant alcoolique est typiquement choisi dans le même groupe que celui de l'étape a), i.e. parmi les monoalcools en C₁-C₄ et les diols.
De préférence, l'élimination du solvant de l'étape d) se fait par évaporation. De préférence, la dilution finale est réalisée dans un diol, de préférence du 1,3-propanediol.

De préférence, entre les étapes c) et d), une étape de décoloration du filtrat obtenu en c) est ajoutée. La décoloration peut se faire par adsorption des pigments présents dans le filtrat sur charbon actif. Cette étape de décoloration peut être suivie d'une étape de filtration du filtrat décoloré obtenu, notamment sur une membrane de 20 µm.

Préférentiellement, l'extrait alcoolique de parties aériennes de *Solidago virgaurea subsp. alpestris* selon l'invention est susceptible d'être obtenu par un procédé comprenant les étapes suivantes :
a) extraction d'un mélange de fleurs, de feuilles et de tiges de *Solidago virgaurea subsp. alpestris*, préalablement séchées et broyées, avec de l'éthanol, à une température comprise entre 40°C et 60°C pendant 2h à 5h ;
b) incubation du mélange obtenu en a) pendant au moins 12h à une température comprise entre 2°C et 6°C ;
c) filtration du mélange incubé obtenu en b), pour obtenir un filtrat ;
   - décoloration du filtrat obtenu en c) par adsorption sur charbon actif ; puis
   - filtration du filtrat décoloré sur une membrane de 20 µm ; et
d) élimination de l'éthanol du filtrat obtenu par évaporation, puis dilution finale dans du 1,3-propanediol.

Avantageusement, l'extrait mis en oeuvre selon l'invention est de couleur claire.

Egalement, ledit extrait se présente sous une forme suffisamment concentrée pour pouvoir être utilisé sans entraîner les problèmes de formulation habituellement rencontrés aux concentrations nécessaires pour obtenir une activité dans les compositions cosmétiques ou dermatologiques sous forme d'émulsion, et sans présenter une couleur foncée, contrairement aux extraits végétaux obtenus par des procédés usuels, lorsqu'ils sont sous forme concentrée.

De ce fait, l'extrait selon l'invention peut être utilisé directement pour la préparation d'une composition cosmétique ou dermatologique.

Selon un aspect ultérieur, l'invention concerne l'utilisation cosmétique d'un extrait alcoolique de parties aériennes de *Solidago virgaurea subsp. alpestris* selon l'invention, en tant qu'antioxydant, et/ou agent dépigmentant, et/ou agent améliorant la microcirculation cutanée, et/ou pour la prévention et/ou l'atténuation d'altérations de la peau dues au vieillissement.

En effet, de manière avantageuse, on a trouvé que l'extrait alcoolique de parties aériennes de *Solidago virgaurea subsp. alpestris* selon l'invention possédait plusieurs activités d'intérêt vis-à-vis de mécanismes physiologiques préventifs ou réparateurs liés aux altérations de la peau, dues notamment au vieillissement.

L'invention concerne donc, plus particulièrement, l'utilisation cosmétique d'un extrait alcoolique de parties aériennes de *Solidago virgaurea subsp. alpestris* selon l'invention en tant qu'agent inhibiteur de la synthèse de mélanine.

On a également trouvé que, de manière avantageuse, l'extrait alcoolique de parties aériennes de *Solidago virgaurea subsp. alpestris* selon l'invention présentait une activité avantageuse à l'égard de la sécrétion de VEGF et de la cytokine IL1α par les kératinocytes. Le VEGF ou facteur de croissance endothélial vasculaire (en anglais « vascular endothelial growth factor ») qui représente dans la peau un facteur majeur de l'angiogénèse cutanée. L'épiderme est une source importante de VEGF, secrété en grande quantité par les kératinocytes en prolifération. L'ARNm du VEGF est exprimé par les kératinocytes normaux, à la fois dans un tissu *in situ* et en culture cellulaire. Il a été montré que le VEGF maintiendrait l'homéostasie des cellules endothéliales et leur capacité à répondre à une stimulation angiogénique, même chez le sujet âgé (Watanabe Y et al., 1997, Oncogene 14 : 2025-2032). D'autre part une diminution du VEGF a été observée suite à une exposition aux radiations UV (Photochem. Photobiol., 1999; 70(4):674-9).

L'invention concerne également l'utilisation d'un extrait alcoolique de parties aériennes de *Solidago virgaurea subsp. alpestris* selon l'invention en tant qu'agent activateur de la sécrétion de VEGF et/ou en tant qu'agent inhibiteur de la sécrétion d'IL1α par les kératinocytes.

L'invention concerne aussi, plus particulièrement, l'utilisation cosmétique d'un extrait alcoolique de parties aériennes de *Solidago virgaurea subsp. alpestris* selon l'invention pour la prévention et/ou l'atténuation d'altérations de la peau dues au vieillissement, notamment par son action inhibitrice des métalloprotéinases, notamment MMP3 et MMP9. L'extrait alcoolique de parties aériennes de *Solidago virgaurea subsp. alpestris* selon l'invention peut donc être utilisé en tant qu'agent inhibiteur de l'activité des métalloprotéinases de la matrice (dénommées MMP pour « matrix metalloprotease »). Les métalloprotéinases de la matrice sont des enzymes qui dégradent la matrice extracellulaire dans le cadre du remodelage physiologique de la peau, mais l'âge et l'exposition aux irradiations UV ont pour conséquence d'augmenter l'activité des MMP spécifiques, notamment la MMP3 et la MMP9. De ce fait, la matrice extracellulaire est dégradée de façon accrue, avec pour résultat l'affaissement des tissus de la peau et la formation de rides (Ageing Res. Rev., 2002, 1(4) :705-20 ; J. Invest. Dermatol, 2001, 117(5):1218-24).

L'invention concerne également, selon un aspect ultérieur, une composition cosmétique ou dermatologique comprenant, dans un véhicule cosmétiquement ou pharmaceutiquement acceptable, un extrait alcoolique de parties aériennes de *Solidago virgaurea subsp. alpestris* selon l'invention. De préférence, ledit extrait est présent dans la composition cosmétique ou dermatologique à raison de 0,001 à 10% en poids total de la composition, en particulier à raison de 0,01 à 10%, de préférence de 0,1 à 10% en poids total de la composition. Ladite composition cosmétique ou dermatologique peut notamment, être adaptée à une application par voie topique.

Avantageusement, ladite composition cosmétique ou dermatologique peut se présenter sous la forme d'une poudre, d'une émulsion, d'une microémulsion, d'une nanoémulsion, d'une suspension, d'une solution d'une lotion, d'une crème, d'un gel aqueux ou hydroalcoolique, d'une mousse, d'un sérum, d'une solution ou d'une dispersion pour aérosol, ou d'une dispersion de vésicules lipidiques.

Dans le cas d'une émulsion, il peut s'agir d'une émulsion eau dans huile ou huile dans eau.

La composition cosmétique ou dermatologique selon l'invention peut comprendre également un solvant choisi en fonction des différents ingrédients et de la forme d'administration.

A titre d'exemples, on peut citer l'eau (de préférence de l'eau déminéralisée), un alcool tel que l'éthanol, ou un éther de diéthylène glycol tel que l'éthoxydiglycol ou l'éther monométhylique de diéthylène glycol.

Ladite composition cosmétique peut également comprendre au moins un additif usuel dans le domaine, tel que par exemple au moins un composé choisi parmi un agent émollient ou humectant, un agent gélifiant et/ou épaississant, un agent tensioactif, une huile, un agent actif, un colorant, un conservateur, un agent antioxydant, un agent actif, une poudre organique ou inorganique, un filtre solaire et un parfum.

Notamment, ladite composition peut contenir :
- Un ou plusieurs agent(s) émollient(s) ou humectant(s), qui peuvent être choisi(s) par exemple parmi la glycérine, les glycols, les silicones hydrosolubles tels que celui vendu sous la dénomination KF6011 (Shin Etsu) et le Jojoba hydrosoluble, tel que celui vendu sous la dénomination Resplanta jojoba (Res pharma).
   Ledit agent émollient ou humectant peut être présent dans la composition à une teneur de l'ordre de 0 à 30%, de préférence 2 à 10% en poids, par rapport au poids total de la composition.
- Un ou plusieurs agent(s) gélifiants(s) et/ou épaississant(s) de la phase aqueuse, choisi par exemple parmi les dérivés cellulosiques, gommes d'origine végétale (guar, caroube, alginates, carraghénanes, pectine), d'origine microbienne (xanthane), les argiles (laponite), les matériaux identifiés par les noms INCI "ammonium acryloyldiméthyltaurate/vp copolymer" et "ammonium acryloyldiméthyl-taurate/beheneth-25 méthacrylate copolymer" (tels que par exemple ceux vendus sous les dénominations Aristoflex AVC et HMB par Clariant).
   Ledit agent gélifiant et/ou épaississant peut être présent dans la composition à une teneur de l'ordre de 0 à 10% en poids, par rapport au poids total de la composition.
- Un ou plusieurs agent(s) tensioactif(s), de préférence non ionique, présent dans une teneur de l'ordre de 0 à 8%, de préférence 0,5 à 3% en poids, par rapport au poids total de la composition.
- Un ou plusieurs corps gras liquide(s) à température ambiante, communément dénommé(s) huiles(s), volatil(s) ou non volatile(s), hydrocarboné(s) ou siliconé(s), linéaire(s), cyclique(s) ou ramifié(s), par exemple, l'isododécane, le cyclopentadimethylsiloxane, les diméthicones, l'isononanoate d'isononyle ou le pentaerythrityl tetraisostéarate, de préférence à raison de 0 à environ 10%, de préférence 0,5 à 5% en poids, par rapport au poids total de la composition.
- Un ou plusieurs agent(s) actif(s), d'origine naturelle ou synthétique, ayant une activité biologique, par exemple choisis parmi les vitamines, les oligo-éléments, l'allantoïne, les protéines végétales et les extraits végétaux.
- Un ou plusieurs colorant(s) hydrosoluble(s) tels que, par exemple, le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine ou la xanthophylle, de préférence à raison de 0 à environ 2% en poids, par rapport au poids total de la composition.

D'autres additifs habituellement utilisés en cosmétique peuvent également être présents dans la composition selon l'invention, notamment des conservateurs, des agents antioxydants ou des parfums bien connus dans le domaine technique.

L'homme du métier est en mesure de choisir, parmi l'ensemble de ces éventuels additifs, aussi bien la nature que la quantité de ceux qui seront ajoutés à la composition, de telle sorte que celle-ci conserve l'ensemble de ses propriétés.

L'invention est illustrée de manière non limitative par les exemples ci-dessous.

### Exemple 1: Préparation d'un extrait alcoolique de parties aériennes de Solidago virgaurea subsp. Alpestris selon l'invention

Un extrait alcoolique de parties aériennes de *Solidago virgaurea subsp. Alpestris* selon l'invention est préparé par un procédé comprenant les étapes suivantes:
a) extraction à l'éthanol des parties aériennes (fleurs, feuilles et tiges) séchées et broyées de *Solidago virgaurea subsp. Alpestris*, à une température de 50°C pendant 3 heures ;
b) incubation du mélange obtenu en a) pendant la nuit à 4°C ;
c) filtration de l'extrait obtenu en b) sur une membrane de 100 µm ;
   - décoloration du filtrat liquide obtenu en c) par adsorption des pigments sur charbon actif (présent en quantité égale à 2% de la plante séchée) ; puis
   - filtration du filtrat décoloré sur une membrane de 20 µm ;
d) élimination de l'éthanol par évaporation, et enfin dilution finale dans du 1,3-propanediol (90% de solvant).

L'extrait ainsi obtenu est appelé « Solidage » dans les exemples 2 à 6 suivants.

### Exemple 2: Test de cytotoxicité de Solidage dans les kératinocytes et mélanocytes humains normaux

### Protocole:

Des kératinocytes et des mélanocytes épidermiques humains normaux (PromoCell) provenant de donneurs juvéniles ont été cultivés dans des plaques 96 puits pendant 24 heures dans un milieu de culture supplémenté (respectivement milieux KGM2 et MGM2 + SupplementMix, Promocell) à 37°C, 5% de CO2. Les cellules ont ensuite été incubées avec différentes concentrations de Solidage dans les milieux de base (sans supplément) pendant 24 heures (kératinocytes) ou dans les milieux supplémentés pendant 5 jours (mélanocytes). La cytotoxicité a été évaluée à l'aide du Cell Titer96 Aqueous One Solution Cell Proliferation Assay (Promega), basé sur la capacité des cellules viables à réduire les sels de tétrazolium incolores/jaunes en dérivé formazan coloré intensément brun. Les cellules ont été incubées avec le tétrazolium à 37°C pendant 30 minutes et l'absorbance du formazan formé a été lue à 490 nm.

### Résultats:

La cytotoxicité de Solidage a été évaluée à différentes concentrations comprises entre 0,1 et 0,0125% (tableau 1 ci-dessous).

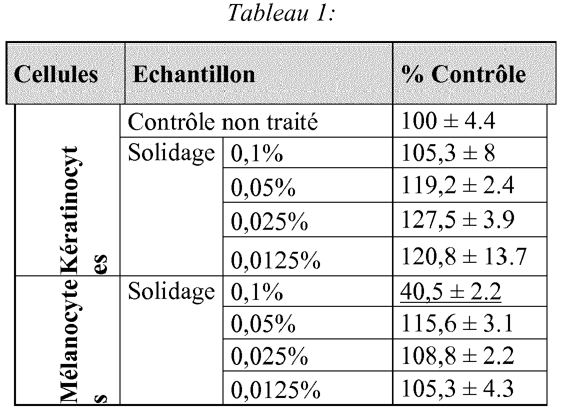

Le Solidage n'est pas toxique pour les kératinocytes à toutes les concentrations testées, alors qu'il est toxique pour les mélanocytes à 0,1%.

### Exemple 3 : Evaluation de l'activité antioxydante de Solidage

### Protocole:

Pour la détermination de la capacité de Solidage à piéger les radicaux libres (hydroxyles, dérivés halogénés, péroxynitrites et radicaux superoxydes), i.e. sa capacité antioxydante, des essais de chemiluminescence dépendants de la pholasin ABEL®1 ont été utilisés (Knight Scientific Limited) selon les instructions du fabricant. Brièvement, diverses concentrations d'ingrédients ont été incubées avec la pholasin, une photoprotéine bien connue qui émet de la lumière lorsqu'elle se lie à des radicaux libres. Du D-Mannitol, de l'albumine, de la vitamine E et de l'acide L-ascorbique ont été utilisés comme témoins positifs pour la détermination de la capacité de l'extrait à piéger respectivement les radicaux hydroxyles, les dérivés halogénés, les péroxynitrites et les superoxydes.

Toutes les déterminations ont été effectuées en 5 réplicates et les valeurs moyennes ont été déterminées. La capacité antioxydante de l'ingrédient est exprimée en pourcentage de réduction du pic de luminescence de Pholasin observé au cours de l'essai en présence et en absence de l'actif ± SD, avec le témoin non traité représenté à 0%.

### Résultats:

Les résultats obtenus sont résumés dans le tableau 2 ci-dessous.

A toutes les concentrations testées, Solidage présente une activité antioxydante importante contre les radicaux hydroxyles, les radicaux halogénés et péroxynitrites, comparable à celle de la concentration la plus élevée testée des composés utilisés comme témoins positifs. Une diminution dose-dépendante du pourcentage de l'activité de capture des superoxydes a été observée avec la diminution de la concentration de Solidage.

**Tableau 2:**

| **Radicaux hydroxyles** | | | | **Halogénés** | | |
|---|---|---|---|---|---|---|
| **Echantillon** | | **Moyenne % inhibition** | | **Echantillon** | | **Moyenne % inhibition** |
| Contrôle non traité | | 0±4,4 | | Contrôle non traité | | 0 ± 1,85 |
| | | | | | | |
| Solidage | 0, 1% | 98,8 ± 0.1 | | Solidage | 0,1% | 99,6 ± 0,02 |
| | 0, 0,05% | 97,2 ± 0,3 | | | 0,05% | 98,9 ± 0,05 |
| | 0, 025% | 94,3 ± 0,1 | | | 0,025% | 96,9 ± 0,04 |
| | 0125% | 89,8 ± 0,03 | | | 0,0125% | 92,8 ± 0,89 |
| | | | | | | |
| D-Mannitol (mM) | 6 | 95,1 ± 0,3 | | Albumine (mg/ml) | 1,25 | 93,9 ± 0,69 |
| | 4 | 92,6 ± 4,7 | | | 1 | 92,1 ± 3,3 |
| | 2 | 82,6 ± 14,8 | | | 0,75 | 88,5 ± 5,7 |
| | 1 | 76,1 ± 14 | | | 0,5 | 82,3 ± 8,9 |
| | 0,8 | 59,9 ± 3,6 | | | 0,25 | 64,9 ± 13,9 |
| | | | | | | |

| **Péroxynitrites** | | | | **Superoxydes** | | |
|---|---|---|---|---|---|---|
| **Echantillon** | | **Moyenne % inhibition** | | **Echantillon** | | **Moyenne % inhibition** |
| Contrôle non traité | | 0 ± 15,3 | | Contrôle non traité | | 0 ± 4,5 |
| | | | | | | |
| Solidage | 0,1% | 115,9 ± 0,7 | | Solidage | 0,1% | 77.7 ± 1 |
| | 0,05% | 118,3 ± 0,1 | | | 0,05% | 54,8 ± 0, |
| | 0,025% | 115,7 ± 0,8 | | | 0,025% | 32,1 ± 2 |
| | 0,0125% | 103,1 ± 0,3 | | | 0,0125% | 32,3 ± 2,9 |
| Vitamine E Analogue (µmol/L) | 5 | 43,9 ± 0 | | Acide L-Ascorbique (µmol/L) | 80 | 82,4 ± 1,8 |
| | 3,125 | 28,4 ± 1,2 | | | 60 | 75,3 ± 1 |
| | 1,875 | 14,5 ± 4,4 | | | 40 | 64,8 ± 0,8 |
| | 1,25 | 10 ± 0 | | | 20 | 43,9 ± 1,4 |
| | 0,625 | 9,2 ± 0 | | | 10 | 26,4 ± 3,5 |

### Exemple 4: Détermination de l'effet du Solidage sur l'activité des métalloprotéases 3 et 9 de la matrice extracellulaire par dosage fluorométrique

### Protocole:

Pour évaluer l'effet de Solidage sur l'activité de MMP3 et MMP9, les enzymes (Biomol SE-109 et SE-244, respectivement) ont été incubées avec différentes concentrations de Solidage, chaque concentration en 5 réplicates. La réaction enzymatique a été induite par l'addition du substrat fluorescent spécifique de chaque enzyme MMP, solubilisé dans du DMSO (Bachem H-2300, M-2055). La réaction enzymatique a été suivie par spectrophotométrie pendant une heure et la fluorescence mesurée après excitation/émission à des longueurs d'onde de 360/460 nm pour MMP3, ou 320/405 nm pour MMP9. Les résultats sont exprimés en moyenne des réplicates ± SD et montrés en % de l'activité des MMP par rapport au témoin non traité, qui représente 100%. Comme témoin, l'EDTA a été utilisé pour l'inhibition de l'activité des MMPs.

### Résultats:

Les enzymes MMPs sont connues pour être impliquées dans la dégradation de la matrice extracellulaire, et ont donc une incidence sur les propriétés mécaniques de la peau associée au vieillissement.

Les inventeurs ont ainsi déterminé la capacité de Solidage à inhiber les activités de MMP3 et MMP9.

Les données résumées dans le tableau 3 indiquent que Solidage inhibe à la fois l'activité de la MMP3 et de la MMP9 à la concentration la plus élevée testée de 0,1%.

Ces résultats montrent que Solidage peut limiter la dégradation de la matrice extracellulaire et ainsi préserver l'intégrité dermique de la peau.

**Tableau 3:**

| | | **Activité enzymatique (%)** | |
|---|---|---|---|
| **Echantillon** | | **MMP3** | **MMP9** |
| Contrôle non traité | | 100 ± 7,8 | 100 ± 2,2 |
| Solidage | 0,1% | 40 ± 3,7 | 60 ± 9,5 |
| EDTA | 5mM | 4,3 ± 5,3 | 0 ± 0,9 |

### Exemple 5: Evaluation de l'effet de Solidage sur la sécrétion de VEGF et d'IL1α par les kératinocytes humains normaux en culture à l'aide de l'essai ELISA

### Protocole:

Les kératinocytes ont été cultivés dans une plaque à 6 puits pendant 24 heures dans du milieu supplémenté (milieu de base KGM2 + SupplementMix, Promocell) avant d'être traités avec diverses concentrations de Solidage en milieu de base pour 24 heures. Les niveaux de VEGF et d'IL1α sécrétés par les kératinocytes dans les milieux de culture ont été déterminés par un dosage ELISA en sandwich classique (Quantikine, R&D Systems) selon les instructions du fabricant. Le niveau de sécrétion de la protéine ciblée a été évalué sur au moins deux donneurs de kératinocytes cultivés en présence ou en absence d'actif. TGFβ et UVB, connu pour augmenter le taux de VEGF et d'IL1α respectivement, ont été utilisés comme témoins positifs.

Les résultats présentés sont des moyennes de triplicates biologiques ± SD et sont représentés en pourcentage par rapport au contrôle (qui représente 100%).

### Résultats:

L'impact de Solidage a été évalué sur le niveau de sécrétion des kératinocytes de deux cibles biologiques spécifiques:
   - IL1α, cytokine bien connue impliquée dans l'inflammation et le vieillissement de la peau, et
   - VEGF, facteur de croissance stimulant la micro-vascularisation, et donc améliorant la fourniture de nutriments et d'oxygène dans la peau.

L'extrait de Solidage inhibe la sécrétion d'IL1α d'une manière dose-dépendante, avec un effet maximal (54,1 ± 14,6%) observé à la concentration de 0,0125%, et les UVB stimulent IL1α (166 ± 20,6%) par rapport à des cellules non traitées (100%). L'évaluation des kératinocytes traités avec 0,1% de Solidage a montré une augmentation de la sécrétion de VEGF (205,4 ± 20,3%) par rapport à des cellules non traitées (100%). Les résultats indiqués issus d'un donneur sont cependant représentatifs des deux donneurs testés.

Il est donc possible de conclure que Solidage est un ingrédient efficace pour l'inhibition de l'IL1α pro-inflammatoire, tandis qu'il stimule VEGF et assure la nutrition de la peau et son oxygénation, ce qui permet de lutter contre le vieillissement de la peau.

### Exemple 6: Détermination de l'effet de Solidage sur la teneur en mélanine dans des mélanocytes humains normaux en culture

### Protocole:

Des mélanocytes épidermiques humains normaux (Promocell) obtenus à partir de deux donneurs de phototype élevé ont été cultivés dans des plaques 96 puits dans du milieu supplémenté (milieu de base MGM2 + SupplementMix, Promocell). Les cellules ont été incubées avec du milieu de croissance contenant diverses concentrations de Solidage (de 0,05 à 0,0625%), pendant 5 jours. Par la suite, le milieu de culture a été éliminé et les cellules lavées avec du PBS (Gibco/Invitrogen). Pour l'extraction de la mélanine intracellulaire, les cellules ont été lysées dans du NaOH 1M, centrifugées à 12 000 tours par minute pendant 5 minutes, et l'absorbance des surnageants clairs a été mesurée à 490 nm. La teneur en mélanine a été normalisée par rapport aux protéines totales par puits à 595 nm (Biorad Protein Assay, Biorad).

Les résultats sont représentés en % du témoin non traité qui est fixé à 100%.

### Résultats:

La mélanine est le chromophore de la peau humaine synthétisé par les mélanocytes de l'épiderme et responsable principalement de la couleur de la peau. L'effet possible de Solidage sur la pigmentation de la peau a été évalué par quantification chimique de mélanine dans les cellules traitées et non traitées.

Le Solidage à la concentration de 0,1% inhibe de 21,9 ± 1,9% la teneur en mélanine des mélanocytes par rapport aux cellules non traitées.

Il ressort de ce test que Solidage module la teneur en mélanine des mélanocytes humains normaux cultivés et peut donc diminuer le niveau de pigmentation de la peau (donc agir comme agent dépigmentant).

### Exemple 7: Détermination de l'effet de Solidage sur la durée de vie réplicative de fibroblastes humains primaires (HDF)

### Protocole:

Des fibroblastes dermiques humains primaires (HDF) ont été isolés à partir de plastie abdominale de trois différents donneurs femmes âgées de 49, 58 et 65 ans, fournies par Evercyte (Vienne, Autriche). Les cellules ont été cultivées à 37°C et 7% de CO2 dans du milieu DMEM F12 / Ham (1:1) de Biochrome (Berlin, Allemagne) supplémenté avec 4 mM de L-glutamine et 10% de sérum de veau foetal. Pour les passages en série, les cellules ont été lavées deux fois avec 1xPBS et incubées avec 0,1% de trypsine / 0,02% d'EDTA pendant 5-8 min. Les cellules qui se détachent ont été remises en suspension dans du milieu contenant Solidage pour atteindre une concentration finale de 0,00625% dans le milieu. Le nombre de cellules viables dans 1 ml de suspension cellulaire a été déterminé automatiquement en utilisant un Vi-CELL XR (Beckman Coulter).

### Résultats:

La culture en continu par passage en série de HDF en présence de Solidage conduit à une prolongation de la durée de vie réplicative des cellules de 10,8% (données non montrées).

### Exemple 8: Détermination de l'effet du traitement continu des cellules HDF avec Solidage sur la transition des fibroblastes papillaires en fibroblastes réticulaires (PRT)

### Protocole:

Les HDF ont été passés en série comme décrit dans l'exemple 7 et été analysés quant à la PRT par inspection visuelle de la morphologie des cellules au microscope optique, et par quantification des marqueurs des phénotypes papillaire (PDPN) et réticulaire (TGM2). Par conséquent, l'ARN a été isolé en utilisant le TRI Reagent (Sigma) selon les instructions du fabricant. L'ARN isolé a été quantifié avec un spectrophotomètre NanoDrop ND-1000 (Thermo Scientific) et 300 ng ont été transcrits en ADNc en utilisant le NCode VILO miRNA cDNA Synthesis Kit (Life Technologies). La PCR quantitative en temps réel (qPCR) a été réalisée sur un RotorGene-6000 Thermocycler (Qiagen) en utilisant HOT FIREPol EvaGreen qPCR Mix Plus (NO ROX) de Solis BioDyne (Tartu, Estonie). L'expression de l'ARNm a été normalisée par rapport à l'expression de GAPDH. Les marqueurs et amorces utilisés sont les suivants :
TGM2: fwd-GGCGAACCACCTGAACAAAC (SEQ ID NO :1) and rev-AGGATGCAAAGAGGAACGCT (SEQ ID NO :2),
PDPN : fwd-GCATCGAGGATCTGCCAACT (SEQ ID NO :3) and rev-CCCTTCAGCTCTTTAGGGCG (SEQ ID NO:4),
GAPDH (contrôle) : fwd-CGACCACTTTGTCAAGCTCA (SEQ ID NO :5) and rev-TGTGAGGAGGGGAGATTCAG (SEQ ID NO:6).

### Résultats:

La culture en continu par passages en série de cellules HDF sans la présence de Solidage conduit à la perte de la morphologie papillaire des HDF, alors qu'elle est maintenue en présence de Solidage (données non montrées).

Ceci est confirmé par le fait que l'expression du marqueur TGM2 (marqueur réticulaire) est plus faible, alors que l'expression du marqueur PDPN (marqueur papillaire) est plus élevée, lorsque les cellules sont cultivées en présence de Solidage, par rapport aux cellules non traitées (données non montrées).

Il est conclu que Solidage retarde la transition du phénotype papillaire au phénotype réticulaire des HDF.

Puisque la peau jeune contient un nombre plus élevé de HDF papillaires, cela suggère encore une fois que Solidage pourrait aider à maintenir un phénotype jeune de la peau.

### Exemple 9: Détermination de l'effet du traitement en continu des cellules HDF avec Solidage sur des marqueurs de sénescence

### Protocole:

Les HDF ont été passés en série comme décrit dans l'exemple 7, et ont été testés pour la sénescence par marquage associé à la β-galactosidase (β-SA-gal). Par conséquent, les cellules ont été lavées deux fois avec 1xPBS et fixées pendant 10 min avec 2% de formaldéhyde / 0,2% de glutaraldéhyde. Les cellules ont été lavées deux fois avec 1xPBS, une fois avec du tampon de coloration (acide 100 mM d'acide citrique / 200 mM Na2HPO4, pH 6,0) et incubées avec une solution de coloration (5 mM de potassium ferricyanure, 5 mM de ferrocyanure de potassium, 2 mM de MgCl2, 1 mg / ml de X-Gal, dilué dans du tampon de coloration) à 37°C pendant 24 heures. Pour chaque puits, 10 photos ont été prises à des endroits aléatoires du puits, et les images d'une expérience ont été randomisées et comptées de façon aveugle par un seul opérateur. En outre, la qPCR a été réalisée comme dans l'exemple 9 en utilisant des amorces des marqueurs de sénescence (P21) et de cellules jeunes (SNEV) suivantes:
p21^{Cip1/WAF1} (CDKN1A) : fwd-GGCGGCAGACCAGCATGACAGATT (SEQ ID NO :7) et rev-GCAGGGGGCGGCCAGGGTAT (SEQ ID NO :8),
SNEV^{hPrp19/hPso4} (PRPF19) : fwd-AACCACGGAGCGCAAGAAG (SEQ ID NO :9) et rev-CGGGGGAAGCAGAAAACAC (SEQ ID NO :10),
Et à nouveau normalisées par rapport aux niveaux d'ARNm de GAPDH.

### Résultats:

la culture en continu par passages en série des HDF en présence de Solidage conduit à une réduction des cellules sénescentes d'environ 50% à tous les temps testés durant le passage en série des cellules (données non montrées). Ceci est reflété par la baisse des niveaux d'ARNm de p21 comme marqueur des cellules sénescentes et par l'augmentation des niveaux d'ARNm de SNEV en tant que marqueur de cellules jeunes (Voglauer et al., 2006).

Cela confirme encore l'activité de Solidage pour retarder l'entrée des cellules en sénescence réplicative.

### Exemple 10: Détermination de l'effet du traitement aigu avec Solidage sur le marqueur de la sénescence dans des HDF sénescentes prématurément sous l'effet d'un stress (HDF SIPS)

### Protocole:

Les cellules ont été ensemencées le jour 0 à 3500 cellules/cm² et traitées aux jours 1-4 et 7-11 avec 100 µM de H2O2 pendant 1 heure par jour, puis récupérées dans un milieu de croissance normal. Les cellules témoins quiescentes ont été ensemencées à la même densité au jour 0 et le milieu a été changé deux fois par semaine. Le traitement avec Solidage a été réalisé immédiatement après le dernier traitement avec H2O2 au jour 11.

Les HDF SIPS ont été testées pour la sénescence par marquage associé à la β-galactosidase (SA-β-gal) comme à l'exemple 10. La qPCR a été effectuée comme dans l'exemple 10. En outre, après contrôle de la qualité de l'ARN isolé avec un Bioanalyzer 2100 (Agilent Technologies) en utilisant un RNA 6000 Nano Kit selon les instructions du fabricant, le séquençage de prochaine génération (NGS) a été réalisé par GATC Biotech (Constance, Allemagne) en utilisant un Illumina HiSeq 2500.

### Résultats:

Un traitement aigu avec Solidage diminue la positivité SA-βGal des HDF SIPS d'environ 44% au jour 4 et 80% au jour 11 (données non montrées). Ceci est accompagné par une diminution de l'ARNm de p21, ainsi que d'une régulation positive de l'ARNm de SNEV. En effet, le principe de l'analyse des composantes principales (PCA) des profils de transcription ARNm du génome total de cellules quiescentes et SIPS, traitées avec Solidage par rapport aux cellules non traitées, montre que Solidage a pour effet de reconvertir le profil du transcriptome à un profil de cellule quiescente. Plus précisément, la transcription d'ARNm de plusieurs facteurs du phénotype sécrétoire associé à la sénéscence (SASP) pro-inflammatoire et dégradant la matrice a été réduite comme illustré à l'aide des exemples de l'IL-11, CXCL8 (IL8), IFI30 (GILT), et CCL2. Pris ensemble, ces résultats suggèrent que Solidage est capable de reconvertir des changements fonctionnels importants qui se produisent lors de l'entrée dans la sénescence cellulaire, et qui contribuent au vieillissement cutané.

### Exemple 11: Détermination de l'effet du traitement aigu avec Solidage sur la survie à long terme des cellules HDF SIPS

### Protocole:

Les cellules ont été ensemencées le jour 0 à 3500 cellules/cm² dans des flacons de culture T75 et traitées aux jours 1-4 et 7-11 avec 100 µM de H2O2 pendant 1 heure par jour, après quoi on les récupère dans un milieu de croissance normal. Les cellules témoins quiescentes ont été ensemencées à la même densité au jour 0 et le milieu a été changé deux fois par semaine. Le traitement avec Solidage a été réalisé immédiatement après le dernier traitement avec H2O2 au jour 11. Le comptage des cellules a été déterminé comme décrit dans l'exemple 7.

### Résultats:

L'exposition des HDF SIPS à Solidage pendant 4 jours ne montre pas d'effet cytotoxique (données non montrées). Toutefois, l'exposition à Solidage pendant plus de 35 jours résulte en une activité d'élimination sélective des cellules sénescentes (SESC) de l'ordre de 30%, tandis que les cellules quiescentes (contrôles) ne montrent pas de quantité significative de mort cellulaire.

Cela indique que l'utilisation à long terme de Solidage pourrait réduire le nombre de cellules sénescentes de façon sélective.

### Exemple 12: Détermination de l'effet du traitement aigu avec Solidage sur la survie à long terme de HDF SIPS

### Protocole:

Des kératinocytes humains primaires dérivés de prépuce néonatal de donneurs individuels (KC) ont été achetés chez CellSystems (Troisdorf, Allemagne) et cultivés dans un milieu de croissance KC (KGM, Clonetics, Gaithersburg, USA) supplémenté avec 0,1 ng/ml d'EGF humain recombinant, 5 µg/ml insuline, 0,5 µg/ml d'hydrocortisone, 0,4% d'extrait d'hypophyse bovine, 50 µg/ml de gentamicine et 50 ng/ml d'amphotéricine B. L'ARN a été isolé en utilisant le système RNeasy 96 (Invitrogen), et 900 ng d'ARN total ont été reverse-transcrits avec le iScript cDNA Synthesis Kit (Biorad). La qPCR a été réalisée en utilisant le LightCycler 480 et le LightCycler 480 SYBR Green I Master (Roche, Bâle, Suisse). L'expression des gènes cibles a été normalisée par rapport à l'expression de la β-2 microglobuline. Les séquences des amorces sont les suivantes (5'-3'):
B2Mf atgagtatgcctgccgtgtg (SEQ ID NO :11); B2Mr: caatccaaatgcggcatct (SEQ ID NO:12);
p16ink4Af: caacgcaccgaatagttacg (SEQ ID NO :13); p16ink4Ar: accagcgtgtccaggaag (SEQ ID NO:14);
MMP1_524f: ggtctctgagggtcaagcag (SEQ ID NO:15); MMP1_720r: ccgcaacacgatgtaagttg (SEQ ID NO:16).

Les cellules sont ensemencées pour les expériences de stress au jour 0 à 1500 cellules/cm² et traitées aux jours 2,5 et 6 avec du Paraquat (PQ) (80 pm) pendant 24 heures. Le traitement avec Solidage a été mené aux jours 1 et 4 sur des cellules non traitées ou traitées avec Paraquat.

### Résultats:

Les KC primaires ont été ensemencés dans des récipients de culture, et traités soit avec PQ, soit avec Solidage, soit avec les deux. Un total de 37500 cellules par récipient de culture a été ensemencé au jour zéro (0), et les nombres de cellules ont été comptés sur 3 et 7 jours de traitement. La moyenne du nombre de cellules comptées pour les cellules contrôles corrigées pour le facteur de division cellulaire était :
- pour les cellules témoins non traitées, à J3 de 720.000, à J7 de 8.295.000 ;
- pour les cellules traitées avec PQ, à J3 de 405.000, à J7 de 546.666 ;
- pour les cellules traitées avec Solidage, à J3 de 825.000, à J7 de 8.400.000 ; et
- pour les cellules traitées avec PQ et Solidage, à J3 de 510.000, à J7 de 1.245.000 cellules.

Ceci indique que Solidage n'empêche pas la diminution de la prolifération cellulaire sous l'effet de stress oxydatif.

Le traitement avec Paraquat, un inducteur bien décrit de la sénescence cellulaire, induit l'expression de MMP1 et p16INK4a.

P16INK4a est un gène marqueur de la sénescence cellulaire et son expression est régulée positivement lorsque les cellules sont en arrêt du cycle cellulaire.

MMP1 est une métalloprotéase matricielle qui est fréquemment sécrétée par des cellules sénescentes.

Quand Solidage a été appliqué en plus de Paraquat, les niveaux d'expression d'ARNm par rapport au gène contrôle (bêta-2-microglobuline) ont été réduits. Ceci indique que Solidage neutralise l'effet du Paraquat sur l'arrêt du cycle cellulaire et sur l'expression de gènes liés à la sénescence.

### Exemple 13: compositions cosmétiques

### 7A - émulsion huile/eau crème gel

| **Nom INCI** | **(% W/W)** |
|---|---|
| Jojoba esters | 1-10 |
| Hydrogenated coconut oil | 1-10 |
| Moringa oil/hydrogenated moringa oil esters (FLORALIPIDS MORINGA BUTTER) | 1-10 |
| Butyrospermum parkii butter (LIPEX SHEASOFT) | 1-10 |
| Camellia kissi seed oil | 1-10 |
| Butyrospernum parkii butter extract (LIPEX SHEA TRIS) | 1-10 |
| Pentaerythrityl stearate/caprate/ caprylate/ adipate (SUPERMOL S-SO) | 0.5-5 |
| Cetyl ethylhexanoate | 1-5 |
| Octyle palmitate | 1-5 |
| Diisostearyl dimer dilinoleate (SCHERCEMOL DISD) | 1-10 |
| Octyldodecyl myristate | 1-5 |
| Hydrogenated lecithin | 0.1-5 |
| Cetearyl alcohol & cetearyl glucoside | 0.1-7 |
| Glyceryl stearate & PEG-100 stearate | 0.1-5 |
| CARBOMER | 0.01-5 |
| BIOSACCHARIDE GUM-1 | 1-10 |
| Methyl methacrylate crosspolymer (MAKIBEADS 150) | 0.1-10 |
| Sodium hyaluronate | 0.01-3 |
| Glycerin | 1-30 |
| Polyquaternium-51 | 1-10 |
| Adenosine | 0.1-0.5 |
| Niacinamide | 0.1-5 |
| Tremella fuciformis polysaccharide | 0.1-5 |
| Palmitoyl Tripeptide-1 & Palmitoyl Tetrapeptide-7 | 1-5 |
| Secale Cereale (Rye) Seed Extract | 1-5 |
| *Solidago virgaurea subsp. Alpestris extract* | 0.01-10 |
| Ascorbyl glucoside | 0.001-5 |
| Glycols (Caprylyl Glycol and/or Pentylene Glycol and/or Butylene Glycol and/or propanediol) | 0,1-10 |
| Water | Qs 100 |

### 7b - émulsion huile/eau crème

| **Nom INCI** | **(% w/w)** |
|---|---|
| Behenyl alcohol | 1-5 |
| Cetyl alcohol | 0.1-5 |
| Phenyl trimethicone | 1-5 |
| Dimethicone & Dimethicone/Vinyl Dimethicone Crosspolymer | 1-30 |
| Ectoin | 0.1-5 |
| PPG-2 myristyl ether propionate | 1-10 |
| Nanofine Titanium Dioxide | 1-20 |
| Zinc Dioxide | 1-20 |
| Diethylamino Hydroxybenzoyl Hexyl Benzoate (Uvinul A+) | 1-5 |
| Bis-Ethylhexyloxyphenol Methoxyphenyl Triazine (Tinosorb S) | 1-5 |
| (Tinosorb M) | 1-5 |
| Ethyl hexyl Methoxycinnamate | 1-7.5 |
| Polysilicone -11 | 1-5 |
| Silica | 1-5 |
| Polymethylsilsesquioxane | 1-5 |
| C20-22 alkyl phosphate & C20-22 alcohols | 0.5-5 |
| Glyceryl stearate & PEG-100 stearate | 0.5-5 |
| sodium acrylate / sodium acryloyldimethyltaurate copolymer | 0.1-5 |
| Hydrogenated starch hydrolysate & maltooligosyl glucoside | 0.1-10 |
| Xanthan Gum | 0,01-2 |
| Agar | 0.1-5 |
| Adenosine | 0.1-0.5 |
| Niacinamide | 0.1-5 |
| Tremella fuciformis polysaccharide | 0.1-5 |
| Palmitoyl Tripeptide-1 & Palmitoyl Tetrapeptide-7 | 1-5 |
| Secale Cereale (Rye) Seed Extract | 1-5 |
| *Solidago virgaurea subsp. Alpestris extract* | 0.01-10 |
| Ascorbyl glucoside | 0.001-5 |
| Water | Qs 100 |

Ces compositions peuvent être appliquées tous les jours, matin et/ou soir, sur la peau.

## Revendications

1. Extrait alcoolique de parties aériennes de *Solidago virgaurea subsp. alpestris*, susceptible d'être obtenu par un procédé comprenant les étapes suivantes :
a) extraction des parties aériennes de *Solidago virgaurea subsp. alpestris*, avec au moins un solvant alcoolique ;
b) incubation du mélange obtenu en a) pendant au moins 10h ;
c) filtration du mélange incubé obtenu en b) ; et
d) élimination du solvant du filtrat obtenu, puis dilution finale dans un autre solvant alcoolique.

2. Extrait alcoolique de parties aériennes de *Solidago virgaurea subsp. alpestris* selon la revendication 1, **caractérisé en ce que** le solvant alcoolique de l'étape a) est un monoalcool comprenant de 1 à 4 atomes de carbone, de préférence l'éthanol.

3. Extrait alcoolique de parties aériennes de *Solidago virgaurea subsp. alpestris* selon la revendication 1 ou 2, **caractérisé en ce que** l'extraction de l'étape a) est réalisée pendant une durée comprise entre 2 et 6 heures, à une température comprise entre 40°C et 60°C.

4. Extrait alcoolique de parties aériennes de *Solidago virgaurea subsp. alpestris* selon l'une des revendications 1 à 3, **caractérisé en ce que** les parties aériennes sont choisies parmi les fleurs, les feuilles, les tiges et leurs mélanges.

5. Extrait alcoolique de parties aériennes de *Solidago virgaurea subsp. alpestris* selon l'une des revendications 1 à 4, **caractérisé en ce que** l'incubation de l'étape b) est réalisée pendant une durée comprise entre 12h et 30h, à une température comprise entre 2°C et 10°C.

6. Extrait alcoolique de parties aériennes de *Solidago virgaurea subsp. alpestris* selon l'une des revendications 1 à 5, **caractérisé en ce que**, entre les étapes c) et d), est ajoutée une étape de décoloration du filtrat obtenu en c), de préférence par adsorption sur charbon actif, suivie d'une étape de filtration du filtrat décoloré obtenu.

7. Extrait alcoolique de parties aériennes de *Solidago virgaurea subsp. alpestris* selon l'une des revendications 1 à 6, **caractérisé en ce que** l'élimination de l'étape d) se fait par évaporation, puis la dilution finale est réalisée dans du 1,3-propanediol.

8. Extrait alcoolique de parties aériennes de *Solidago virgaurea subsp. alpestris* selon l'une des revendications 1 à 7, **caractérisé en ce qu'**il est susceptible d'être obtenu par un procédé comprenant les étapes suivantes :
a) extraction d'un mélange de fleurs, de feuilles et de tiges de *Solidago virgaurea subsp. alpestris*, préalablement séchées et broyées, avec de l'éthanol, à une température comprise entre 40°C et 60°C pendant 2h à 5h ;
b) incubation du mélange obtenu en a) pendant au moins 12h à une température comprise entre 2°C et 6°C ;
c) filtration du mélange incubé obtenu en b), pour obtenir un filtrat ;
- décoloration du filtrat obtenu en c) par adsorption sur charbon actif ; puis
- filtration du filtrat décoloré sur une membrane de 20 µm ; et
d) élimination de l'éthanol du filtrat obtenu par évaporation, puis dilution finale dans du 1,3-propanediol.

9. Procédé d'extraction de parties aériennes de *Solidago virgaurea subsp. alpestris*, comprenant les étapes suivantes :
a) extraction des parties aériennes de *Solidago virgaurea subsp. alpestris*, avec au moins un solvant alcoolique ;
b) incubation du mélange obtenu en a) pendant au moins 10h ;
c) filtration du mélange incubé obtenu en b) ;
d) élimination du solvant du mélange obtenu en c), puis dilution finale dans un autre solvant alcoolique.

10. Utilisation cosmétique d'un extrait alcoolique de parties aériennes de *Solidago virgaurea subsp. alpestris* selon l'une quelconque des revendications 1 à 8 pour la prévention et/ou l'atténuation d'altérations de la peau dues au vieillissement.

11. Utilisation cosmétique d'un extrait alcoolique de parties aériennes de *Solidago virgaurea subsp. alpestris* selon l'une quelconque des revendications 1 à 8 comme agent dépigmentant.

12. Utilisation cosmétique d'un extrait alcoolique de parties aériennes de *Solidago virgaurea subsp. alpestris* selon l'une quelconque des revendications 1 à 8 comme agent améliorant la microcirculation cutanée et/ou agent de régénération de l'épiderme, pour améliorer la couleur et/ou l'éclat et/ou l'uniformité du teint de la peau ; pour prévenir l'apparition d'une peau terne et/ou sans éclat et apporter à la peau un teint régulier et éclatant.

13. Utilisation cosmétique d'un extrait alcoolique de parties aériennes de *Solidago virgaurea subsp. alpestris* selon l'une quelconque des revendications 1 à 8 comme antioxydant.

14. Composition cosmétique ou dermatologique comprenant, dans un véhicule cosmétiquement ou pharmaceutiquement acceptable, un extrait alcoolique de parties aériennes de *Solidago virgaurea subsp. alpestris* selon l'une quelconque des revendications 1 à 8.

15. Composition cosmétique ou dermatologique selon la revendication 14, **caractérisée en ce qu'**elle est adaptée à une application par voie topique.

## Patentansprüche

1. Alkoholischer Extrakt der oberirdischen Teile von *Solidago virgaurea subsp. alpestris*, welcher durch ein Verfahren erhalten werden kann, welches die folgenden Schritte umfasst:
a) Extraktion der oberirdischen Teile von *Solidago virgaurea subsp. alpestris* mit wenigstens einem alkoholischen Lösungsmittel;
b) Inkubieren der in a) erhaltenen Mischung während wenigstens 10 h;
c) Filtrieren der in b) erhaltenen inkubierten Mischung; und
d) Entfernen des Lösungsmittels aus dem erhaltenen Filtrat, dann endgültige Verdünnung in einem anderen alkoholischen Lösungsmittel.

2. Alkoholischer Extrakt der oberirdischen Teile von *Solidago virgaurea subsp. alpestris* nach Anspruch 1, **dadurch gekennzeichnet, dass** das alkoholische Lösungsmittel in Schritt a) ein einwertiger Alkohol ist, der 1 bis 4 Kohlenstoffatome umfasst, bevorzugt Ethanol.

3. Alkoholischer Extrakt der oberirdischen Teile von *Solidago virgaurea subsp. alpestris* nach Anspruch 1 oder 2, **dadurch gekennzeichnet, dass** die Extraktion in Schritt a) über eine Dauer von 2 bis 6 Stunden bei einer Temperatur von 40 bis 60 °C durchgeführt wird.

4. Alkoholischer Extrakt der oberirdischen Teile von *Solidago virgaurea subsp. alpestris* nach einem der Ansprüche 1 bis 3, **dadurch gekennzeichnet, dass** die oberirdischen Teile gewählt sind aus Blüten, Blättern, Stängeln und deren Mischungen.

5. Alkoholischer Extrakt der oberirdischen Teile von *Solidago virgaurea subsp. alpestris* nach einem der Ansprüche 1 bis 4, **dadurch gekennzeichnet, dass** das Inkubieren in Schritt b) über eine Dauer von 12 bis 30 h bei einer Temperatur von 2 bis 10 °C durchgeführt wird.

6. Alkoholischer Extrakt der oberirdischen Teile von *Solidago virgaurea subsp. alpestris* nach einem der Ansprüche 1 bis 5, **dadurch gekennzeichnet, dass** zwischen den Schritten c) und d) ein Schritt der Entfärbung des in c) erhaltenen Filtrats hinzugefügt wird, bevorzugt durch Absorption auf Aktivkohle, gefolgt von einem Schritt des Filtrierens des erhaltenen entfärbten Filtrats.

7. Alkoholischer Extrakt der oberirdischen Teile von *Solidago virgaurea subsp. alpestris* nach einem der Ansprüche 1 bis 6, **dadurch gekennzeichnet, dass** die Entfernung in Schritt d) durch Verdampfen erfolgt; anschließend wird die endgültige Verdünnung in 1,3-Propanediol hergestellt.

8. Alkoholischer Extrakt der oberirdischen Teile von *Solidago virgaurea subsp. alpestris* nach einem der Ansprüche 1 bis 7, **dadurch gekennzeichnet, dass** er durch ein Verfahren erhalten wird, welches die folgenden Schritte umfasst:
a) Extraktion einer Mischung aus zuvor getrockneten und zerkleinerten Blüten, Blättern und Stängeln von *Solidago virgaurea subsp. alpestris* mit Ethanol bei einer Temperatur von 40 bis 60 °C während 2 bis 5 h;
b) Inkubieren der in a) erhaltenen Mischung während wenigstens 12 h bei einer Temperatur von 2 bis 6 °C;
c) Filtrieren der in b) erhaltenen inkubierten Mischung zum Erhalten eines Filtrats;
- Entfärben des in c) erhaltenen Filtrats durch Absorption auf Aktivkohle; dann
- Filtrieren des entfärbten Filtrats auf einer 20 µm-Membran; und
d) Entfernen des Ethanols aus dem durch Verdampfen erhaltenen Filtrat, dann endgültige Verdünnung in 1,3-Propanediol.

9. Extraktionsverfahren der oberirdischen Teile von *Solidago virgaurea subsp. alpestris*, welches die folgenden Schritte umfasst:
a) Extraktion der oberirdischen Teile von *Solidago virgaurea subsp. alpestris* mit wenigstens einem alkoholischen Lösungsmittel;
b) Inkubieren der in a) erhaltenen Mischung während wenigstens 10 h;
c) Filtrieren der in b) erhaltenen inkubierten Mischung; und
d) Entfernen des Lösungsmittels aus der in c) erhaltenen Mischung, dann endgültige Verdünnung in einem anderen alkoholischen Lösungsmittel.

10. Kosmetische Verwendung eines alkoholischen Extrakts der oberirdischen Teile von *Solidago virgaurea subsp. alpestris* nach einem der Ansprüche 1 bis 8 zur Prävention und/oder Abschwächung von Hautveränderungen aufgrund von Alterung.

11. Kosmetische Verwendung eines alkoholischen Extrakts der oberirdischen Teile von *Solidago virgaurea subsp. alpestris* nach einem der Ansprüche 1 bis 8 als Depigmentierungsmittel.

12. Kosmetische Verwendung eines alkoholischen Extrakts der oberirdischen Teile von *Solidago virgaurea subsp. alpestris* nach einem der Ansprüche 1 bis 8 als Mittel zur Verbesserung der Mikrozirkulation der Haut und/oder als Mittel zur Regeneration der Epidermis, zur Verbesserung der Farbe und/oder der Leuchtkraft und/oder der Gleichmäßigkeit des Teints der Haut; zur Prävention von müder und/oder matter Haut und um der Haut einen gleichmäßigen und leuchtenden Teint zu verleihen.

13. Kosmetische Verwendung eines alkoholischen Extrakts der oberirdischen Teile von *Solidago virgaurea subsp. alpestris* nach einem der Ansprüche 1 bis 8 als Antioxidans.

14. Kosmetische oder dermatologische Zusammensetzung, welche in einem kosmetisch oder pharmazeutisch akzeptablen Träger einen alkoholischen Extrakt der oberirdischen Teile von *Solidago virgaurea subsp. alpestris* nach einem der Ansprüche 1 bis 8 umfasst.

15. Kosmetische oder dermatologische Zusammensetzung nach Anspruch 14, **dadurch gekennzeichnet, dass** sie für eine topische Anwendung angepasst ist.

## Claims

1. Alcoholic extract of aerial parts *of Solidago virgaurea subsp. alpestris*, obtainable by a method comprising the following steps:
a) extracting the aerial parts of *Solidago virgaurea subsp. alpestris*, with at least one alcoholic solvent;
b) incubating the mixture obtained in a) for at least 10h;
c) filtering the incubated mixture obtained in b); and
d) removing the solvent from the filtrate obtained, and then final dilution in another alcoholic solvent.

2. Alcoholic extract of aerial parts of *Solidago virgaurea subsp. alpestris* according to Claim 1, **characterized in that** the alcoholic solvent in step a) is a monohydric alcohol comprising from 1 to 4 carbon atoms, preferably ethanol.

3. Alcoholic extract of aerial parts *of Solidago virgaurea subsp. alpestris* according to Claim 1 or 2, **characterized in that** the extraction in step a) is carried out for a length of time between 2 and 6 hours, at a temperature between 40°C and 60°C.

4. Alcoholic extract of aerial parts of *Solidago virgaurea subsp. alpestris* according to one of Claims 1 to 3, **characterized in that** the aerial parts are selected from the flowers, leaves, stems and mixtures thereof

5. Alcoholic extract of aerial parts of *Solidago virgaurea subsp. alpestris* according to one of Claims 1 to 4, **characterized in that** the incubation in step b) is carried out for a length of time between 12h and 30h, at a temperature between 2°C and 10°C.

6. Alcoholic extract of aerial parts of *Solidago virgaurea subsp. alpestris* according to one of Claims 1 to 5, **characterized in that**, between steps c) and d), a step of bleaching of the filtrate obtained in c) is added, preferably by adsorption on activated charcoal, followed by a step of filtration of the decoloured filtrate obtained.

7. Alcoholic extract of aerial parts of *Solidago virgaurea subsp. alpestris* according to one of Claims 1 to 6, **characterized in that** the removal in step d) takes place by evaporation, then final dilution is carried out in 1,3-propanediol.

8. Alcoholic extract of aerial parts of *Solidago virgaurea subsp. alpestris* according to one of Claims 1 to 7, **characterized in that** it is obtainable by a method comprising the following steps:
a) extracting a mixture of flowers, leaves and stems *of Solidago virgaurea subsp. alpestris*, previously dried and ground, with ethanol, at a temperature between 40°C and 60°C for 2h to 5h;
b) incubating the mixture obtained in a) for at least 12h at a temperature between 2°C and 6°C;
c) filtering the incubated mixture obtained in b), to obtain a filtrate;
- bleaching the filtrate obtained in c) by adsorption on activated charcoal; then
- filtering the decoloured filtrate on a 20-µm membrane; and
d) removing the ethanol from the filtrate obtained by evaporation, and then final dilution in 1,3-propanediol.

9. Method for extracting aerial parts of *Solidago virgaurea subsp. alpestris*, comprising the following steps:
a) extracting the aerial parts of *Solidago virgaurea subsp. alpestris* with at least one alcoholic solvent;
b) incubating the mixture obtained in a) for at least 10h;
c) filtering the incubated mixture obtained in b);
d) removing the solvent from the mixture obtained in c), and then final dilution in another alcoholic solvent.

10. Cosmetic use of an alcoholic extract of aerial parts of *Solidago virgaurea subsp. alpestris* according to any one of Claims 1 to 8 for preventing and/or attenuating changes to the skin due to ageing.

11. Cosmetic use of an alcoholic extract of aerial parts of *Solidago virgaurea subsp. alpestris* according to any one of Claims 1 to 8 as a depigmenting agent.

12. Cosmetic use of an alcoholic extract of aerial parts of *Solidago virgaurea subsp. alpestris* according to any one of Claims 1 to 8 as an agent for improving the microcirculation in the skin and/or an agent for regenerating the epidermis, for improving the colour and/or radiance and/or uniformity of the complexion of the skin; for preventing the appearance of skin that is dull and/or without radiance and endowing the skin with an even and radiant complexion.

13. Cosmetic use of an alcoholic extract of aerial parts of *Solidago virgaurea subsp. alpestris* according to any one of Claims 1 to 8 as an antioxidant.

14. Cosmetic or dermatological composition comprising, in a cosmetically or pharmaceutically acceptable vehicle, an alcoholic extract of aerial parts of *Solidago virgaurea subsp. alpestris* according to any one of Claims 1 to 8.

15. Cosmetic or dermatological composition according to Claim 14, **characterized in that** it is suitable for application by the topical route.


### SEQUENCE LISTING

<110> CHANEL et al
<120> Extrait alcoolique de parties aériennes de Solidago virgaurea subsp. alpestris, son procédé d'obtention, et composition cosmétique ou dermatologique le contenant
<130> BFF150211
<160> 16
<170> PatentIn version 3.5
<210> 1
   <211> 20
   <212> DNA
   <213> Artificial Sequence
<220>
   <223> primer forward TGM2
<400> 1
   ggcgaaccac ctgaacaaac 20
<210> 2
   <211> 20
   <212> DNA
   <213> Artificial Sequence
<220>
   <223> primer reverse TGM2
<400> 2
   aggatgcaaa gaggaacgct 20
<210> 3
   <211> 20
   <212> DNA
   <213> Artificial Sequence
<220>
   <223> primer forward PDPN
<400> 3
   gcatcgagga tctgccaact 20
<210> 4
   <211> 20
   <212> DNA
   <213> Artificial Sequence
<220>
   <223> primer reverse PDPN
<400> 4
   cccttcagct ctttagggcg 20
<210> 5
   <211> 20
   <212> DNA
   <213> Artificial Sequence
<220>
   <223> primer forward GAPDH
<400> 5
   cgaccacttt gtcaagctca 20
<210> 6
   <211> 20
   <212> DNA
   <213> Artificial Sequence
<220>
   <223> primer reverse GAPDH
<400> 6
   tgtgaggagg ggagattcag 20
<210> 7
   <211> 24
   <212> DNA
   <213> Artificial Sequence
<220>
   <223> primer forward CDKN1A
<400> 7
   ggcggcagac cagcatgaca gatt 24
<210> 8
   <211> 20
   <212> DNA
   <213> Artificial Sequence
<220>
   <223> primer reverse CDKN1A
<400> 8
   gcagggggcg gccagggtat 20
<210> 9
   <211> 19
   <212> DNA
   <213> Artificial Sequence
<220>
   <223> primer forward PRPF19
<400> 9
   aaccacggag cgcaagaag 19
<210> 10
   <211> 19
   <212> DNA
   <213> Artificial Sequence
<220>
   <223> primer reverse PRPF19
<400> 10
   cgggggaagc agaaaacac 19
<210> 11
   <211> 20
   <212> DNA
   <213> Artificial Sequence
<220>
   <223> primer forward B2M
<400> 11
   atgagtatgc ctgccgtgtg 20
<210> 12
   <211> 19
   <212> DNA
   <213> Artificial Sequence
<220>
   <223> primer reverse B2M
<400> 12
   caatccaaat gcggcatct 19
<210> 13
   <211> 20
   <212> DNA
   <213> Artificial Sequence
<220>
   <223> primer forward p16ink4A
<400> 13
   caacgcaccg aatagttacg 20
<210> 14
   <211> 18
   <212> DNA
   <213> Artificial Sequence
<220>
   <223> primer reverse p16ink4A
<400> 14
   accagcgtgt ccaggaag 18
<210> 15
   <211> 20
   <212> DNA
   <213> Artificial Sequence
<220>
   <223> primer forward MMP1_524
<400> 15
   ggtctctgag ggtcaagcag 20
<210> 16
   <211> 20
   <212> DNA
   <213> Artificial Sequence
<220>
   <223> primer reverse MMP1_720
<400> 16
   ccgcaacacg atgtaagttg 20